# EUROPEAN PATENT APPLICATION

(11) **EP 4 806 542 A1**
(43) Date of publication of application: **16.09.2026**
(21) Application number: 26162235.1
(22) Date of filing: 04.03.2026
(51) Int. Cl.: B01D 61/22

(54) **DETERMINATION APPARATUS, DETERMINATION METHOD, DETERMINATION PROGRAM, AND WATER TREATMENT SYSTEM**

(30) Priority: 11.03.2025 JP 2025038124
(71) Applicant: Yokogawa Electric Corporation, Musashino-shi, Tokyo 180-8750 (JP)
(72) Inventor: MATSUI, Yasuhiro, Musashino-shi, Tokyo, 180-8750 (JP)
(74) Representative: Winter, Brandl - Partnerschaft mbB

(57) **Abstract**

A determination apparatus (74) acquires a first quantity that is a quantity of viruses in membrane filtration supplied water that is supplied to a membrane filtration apparatus and a second quantity that is a quantity of viruses in membrane filtration permeated water after membrane filtration performed by the membrane filtration apparatus. The determination apparatus (74) determines whether or not to perform maintenance of the membrane filtration apparatus based on the first quantity and the second quantity.

## Description

### BACKGROUND

The present disclosure relates to a determination apparatus, a determination method, a determination program, and a water treatment system.

A water treatment system (water treatment device) for producing industrial water, drinking water, and ultrapure water by performing advanced water treatment driven by autonomous optimization on recycled sewage water has been known. In recent years, autonomous operation of water treatment systems using artificial intelligence (AI) has been achieved.
Patent Literature 1: Japanese Laid-open Patent Publication No. 2020-025943
Patent Literature 2: Japanese Laid-open Patent Publication No. 2020-065964
Patent Literature 3: Japanese Laid-open Patent Publication No. 2019-010614
Patent Literature 4: Japanese Laid-open Patent Publication No. 2023-121593

The conventional technique however has a problem in that it is not possible to grasp performance of a water treatment system efficiently in some cases.

For example, the conventional water treatment system does not have any function for grasping performance such as blocking performance relating to acute toxicity and low virulence.

An object of the present disclosure is to grasp performance of a water treatment system efficiently.

### SUMMARY

According to an aspect of an embodiment, a determination apparatus includes a processor that executes acquiring a first quantity that is a quantity of viruses in membrane filtration supplied water that is supplied to a membrane filtration apparatus and a second quantity that is a quantity of viruses in membrane filtration permeated water after membrane filtration performed by the membrane filtration apparatus, and determining whether or not to perform maintenance of the membrane filtration apparatus based on the first quantity and the second quantity.

According to an aspect of an embodiment, a determination method carried out by a computer includes acquiring a first quantity that is a quantity of viruses in membrane filtration supplied water that is supplied to a membrane filtration apparatus and a second quantity that is a quantity of viruses in membrane filtration permeated water after membrane filtration performed by the membrane filtration apparatus, and determining whether or not to perform maintenance of the membrane filtration apparatus based on the first quantity and the second quantity.

According to an aspect of an embodiment, a determination program causes a computer to acquire a first quantity that is a quantity of viruses in membrane filtration supplied water that is supplied to a membrane filtration apparatus and a second quantity that is a quantity of viruses in membrane filtration permeated water after membrane filtration performed by the membrane filtration apparatus, and determine whether or not to perform maintenance of the membrane filtration apparatus based on the first quantity and the second quantity.

According to an aspect of an embodiment, a water treatment system includes an equipment system including each water treatment device that executes a water treatment process and a control device that executes control on the each water treatment device, and a management system that includes a management apparatus that determines content of control on the water treatment process using a virtual system having virtualized the each water treatment device and that controls the water treatment process of the equipment system based on the content of control via the control apparatus of the equipment system, wherein the water treatment device includes a determination apparatus including a processor that acquires a first quantity that is a quantity of viruses in membrane filtration supplied water that is supplied to a membrane filtration apparatus and a second quantity that is a quantity of viruses in membrane filtration permeated water after membrane filtration performed by the membrane filtration apparatus, and determines whether or not to perform maintenance of the membrane filtration apparatus based on the first quantity and the second quantity.

The above and other objects, features, advantages and technical and industrial significance of this invention will be better understood by reading the following detailed description of presently preferred embodiments of the invention, when considered in connection with the accompanying drawings.

### BRIEF DESCRIPTION OF THE DRAWINGS

FIG. 1 is a diagram illustrating an overall configuration of a water treatment system according to a first embodiment;
FIG. 2 is a diagram illustrating an architecture of a water treatment system of a referential technique;
FIG. 3 is a functional block diagram illustrating a functional configuration of the water treatment system according to the first embodiment;
FIG. 4 is a functional block diagram illustrating a functional configuration of a measurement apparatus according to the first embodiment;
FIG. 5 is a flowchart illustrating a flow of a process that is executed by an equipment system;
FIG. 6 is a flowchart illustrating a flow of a process that is executed by a management system;
FIG. 7 is a flowchart illustrating a flow of a process that is executed by a measurement apparatus;
FIG. 8 is a diagram illustrating an architecture of a water treatment system according to a second embodiment;
FIG. 9 is a diagram illustrating an architecture of a water treatment system according to a third embodiment; and
FIG. 10 is a diagram illustrating an example of a hardware configuration.

### DESCRIPTION OF THE EMBODIMENTS

A determination apparatus, a determination method, a determination program, and a water treatment system disclosed by the present application will be described in detail below according to the accompanying drawings. Note that the embodiment does not limit the disclosure. The same elements are denoted with the same reference numerals and redundant description will be omitted as appropriate. It is possible to combine each embodiment as long as no inconsistency is caused.

### About Water Treatment System

A water treatment system used in a water purification plant and the like is configured by a combination of systems (level 0) having four types of functions that directly have an effect on water not to be treated. Note that the devices that execute the four types of functions include: a device (membrane) that executes treatment of physically filtering water; a device (UV/Cl/Ozone) that executes treatment of chemically oxidizing or disinfecting water; devices (mechanical components) that control conveyance of water, such as a pipe, a pump, and a valve; and a device (control H&S) including a measurement device and software necessary to control quality of water, an amount of water, a water temperature, a water pressure, etc.

In such a water treatment system, safe operations are performed by executing various instructions from a programmable logic controller (PLC) located at a higher level than each of the above-described devices to each of the devices located at a lower level according to a logic in each unit of control using the PLC.

In recent water treatment systems, operation control using artificial intelligence (AI) has been achieved. For example, in the operation control using AI, terminal devices that are systems at Level 0 are put together in stages gradually to a system at a higher level constitutively and also as input/output data and, lastly, an AI engine (machine learning model) simulates human thought and derives an optimized solution. According to the solution, an instruction is transmitted to each device.

The above-described water treatment system, however, is a system configured at Level 1, Level 2, or lower and is controlled by sequence control by the PLC and PID (P: proportion, I: integration, and D: differentiation) control is commonly used as the sequence control. In this case, while it is possible to assign an operation value corresponding to a load that the system encounters and execute a command to stop the operation and to avoid a trouble, automated operation using AI is not taken into consideration for the design and thus it it hard to say that optimal automated operation has been achieved.

In contrast to this, according to the water treatment system of the embodiment, it is possible to achieve optimal automated operation.

### First Embodiment

### Overall Configuration (Architecture)

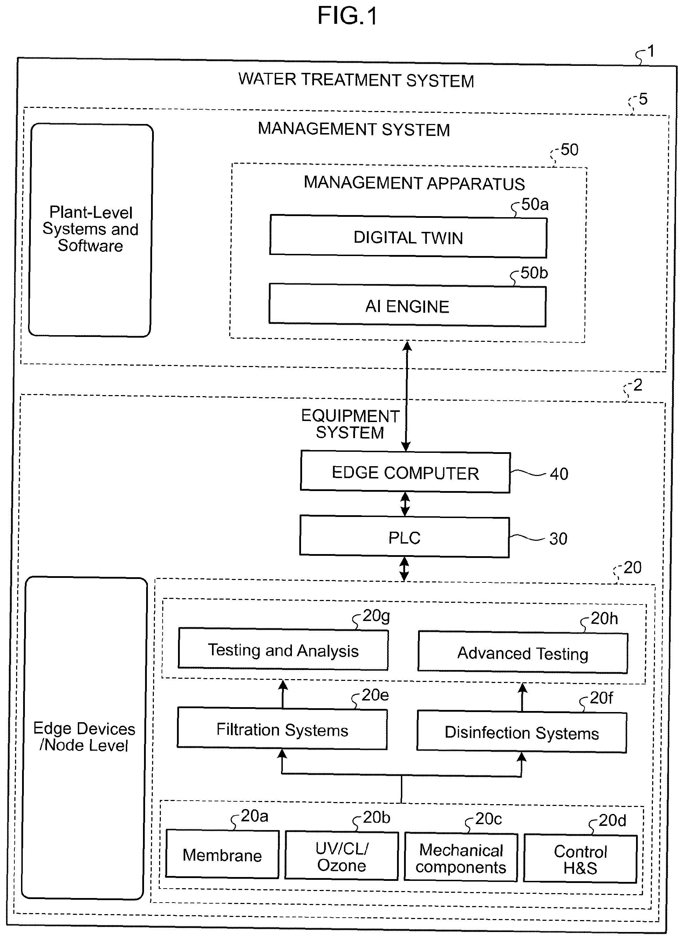

FIG. 1 is a diagram illustrating an overall configuration of a water treatment system according to a first embodiment. A water treatment system 1 illustrated in FIG. 1 is an example of a system that generates drinking water from wastewater, such as sewage and rainwater, by executing advanced water treatment. The water treatment system 1 is roughly classified into two categories. One is a management system 5 corresponding to plant-level-system and software and the other is an equipment system 2 corresponding to edge device/node level constituting actual equipment (actual system).

The equipment system 2 includes each water treatment device that executes a water treatment process and is connected to the management system 5 via a network N. Note that, various networks, such as a dedicated line, a local area network (LAN), a virtual local area network (VLAN), and the Internet can be employed as the network N.

The equipment system 2 includes a water treatment device group 20, a PLC 30, and an edge computer 40. The water treatment device group 20 includes edge devices at a node level. Here, the edge devices at a node level include membranes 20a, a UV/Cl/Ozone 20b, mechanical components 20c, control H&S 20d, filtration systems 20e, disinfection systems 20f, testing and analysis 20g, and an advanced testing 20h. Note that these devices are modularized and each of the devices is controllable.

The membranes 20a are devices that execute treatment of physically filtering water. The UV/Cl/Ozone 20b is a device that executes treatment of performing chemical accelerated oxidation or disinfection on water. The mechanical components 20c are devices that control conveyance of water, such as pipes, pumps, and valves. The control H&S 20d is a device including a measurement device and software necessary to control quality of water, an amount of water, a water pressure, a water temperature, etc.

The filtration systems 20e is a device (system) that is configured by any combination of the above-described devices 20a to 20d and that executes filtration on water. The disinfection systems 20f are devices (system) that are configured by any combination of the devices 20a to 20d and that execute disinfection on water using ultraviolet rays, heat, and the like. The testing and analysis 20g is a device that executes general testing and analysis of pH, electric conductivity, turbidity, water temperature, ultraviolet absorbance, chemical oxygen demand (COD), nitrogen (ammonia nitrogen, nitrate nitrogen, and total nitrogen), and the like, whereas the filtration systems 20e and the disinfection system 20f are devices (systems) that perform physical or chemical water treatment or a water treatment process thereof. The testing and analysis 20g has functions of measurement related to mechanical process management (e.g., on/off of pump and open/close of valve) and operation quality management (e.g., amount of water, water pressure, and quality of water) and transmission and transfer of an analog or digital signal. The advanced testing 20h is a device that executes advanced testing and analysis, such as polymerase chain reaction (PCR), total organic carbon (TOC), and adenosine tri-phosphate (ATP), on water that has been subjected to any one of or both of filtration and disinfection by any one of or both of the filtration system 20e and the disinfection systems 20f. The advanced testing 20h has functions of measurement related to management of quality of water that is safe to human body in operation quality management and transmission and transfer of an analog or digital signal.

The PLC 30 is an example of a computer that performs the above-described sequence control on each of the devices at Level 0, Level 1, Level 2, or lower. The PLC 30 executes various instructions to each water treatment device accordance to an instruction from the edge computer 40 located at a higher level.

The edge computer 40 executes operation control on the water treatment process in the equipment system 2. For example, the edge computer 40 receives data on a control status, content of control, a result of control, a treatment status, and the like, of the water treatment process from the PLC 30, executes simulation using the data and prediction using a machine learning model using the data, and acquires a result of predicting a state of the water treatment process, and the like. The edge computer 40 then notifies the PLC 30 of the content of control in order to, for example, improve the water treatment process, improve the quality, reduce costs, and adjust a volume of production by using the result of predicting a state of the water treatment process.

The management system 5 is an example of a computer that controls the water treatment process by controlling the entire equipment system 2 and includes a plant-level system and software. The management system 5 includes a management apparatus 50. The management system 5 includes a digital twin 50a that generates a virtual module (virtual system) simulating a system configured at a level of actual equipment and an AI engine 50b that causes the digital twin 50a to operate and that controls the equipment system 2, which is the actual equipment. Note that the management system 5 can be implemented by a physical machine including a memory and a processor, cloud computing, and the like. The management apparatus 50 can also be implemented by a physical machine, or can be implemented by a virtual machine, a container, and the like, using virtual technology.

The digital twin 50a collects various types of information from the equipment system 2 and each apparatus in physical space and, using the various types of collected information, reproduces the physical space in virtual space. In other words, the digital twin 50a virtually configures a virtual system that simulates the same variation as that of the equipment system 2, which is actual equipment. The virtual system simulated by the digital twin 50a includes elements of each water treatment device and a virtual terminal device or a virtual module obtained by combining the elements. The virtual module includes a virtual water treatment device corresponding to each water treatment device of the equipment system 2, which is actual equipment.

In the virtual system, it is possible to set and update a parameter enabling direct or indirect calculation of the causal relation between input and output to and from the virtual terminal device or the virtual module based on preliminarily collected data on actual equipment and data sequentially transmitted from the actual equipment. The AI engine 50b to be described below sets and updates the parameter.

Note that the digital twin 50a is also able to form a virtual system by freely combining virtual water treatment devices of a module or virtual modules.

Regarding the virtual system on the digital twin 50a, the AI engine 50b is able to derive a virtual operation condition optimal for various environments and calculate an input/output value by collating the virtual module with not only a configuration simulating the actual equipment but also a module including individual virtual water treatment devices. The AI engine 50b outputs the optimal virtual operation condition and the input/output value to an administrator and the like or notifies the edge computer 40 of the condition and the value to execute a parameter update.

Furthermore, on the virtual system implemented by the digital twin 50a, the AI engine 50b performs simulation on the virtual system for sudden variation and specifies an optimal system configuration. The AI engine 50b then outputs a change to the optimal system configuration to the administrator and the like and updates the parameter that the edge computer 40 uses for simulation and the like.

Furthermore, the AI engine 50b can also use, for example, a machine learning model using deep learning and the like. Note that the management apparatus 50 may be implemented by a cloud system or may be disposed in a module. The management apparatus 50 may have a function of displaying a schematic diagram representing a configuration of a virtual system, a schematic diagram representing a configuration of an actual system, and input/output values and parameters of each water treatment device, each module, each virtual water treatment device, and each water treatment module.

### Description of Reference Technique and Problems

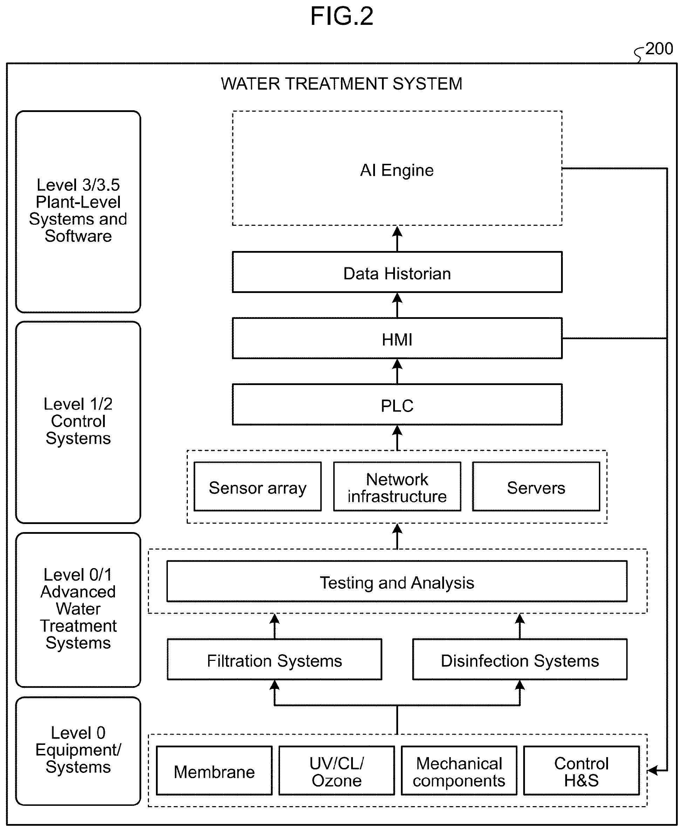

Here, a commonly used water treatment system will be described as reference technique. FIG. 2 is a diagram illustrating the architecture of a water treatment system 200 according to the reference technique. As illustrated in Figure 2, the water treatment system 200, such as a water purification plant, includes apparatuses and systems at Level 0, advanced water treatment purification systems at Level 0 and Level 1, control systems at Level 1 and Level 2, and plant-level systems and software at Level 3 and Level 3.5.

In order to control the entire system in accordance with water to be treated, an installation environment, and a purpose of treatment, the water treatment system 200 described above is constructed in a configuration in which the systems at Level 0 are combined and modularized as systems at Level 1 and Level 2 such as the filtration systems and the disinfection systems.

Furthermore, in the water treatment system 200, testing and analysis are performed on each of the modularized systems at Level 0 or systems at Level 0 and Level 1. The systems at Level 1 and Level 2 that are systems at a higher level include a sensor array, network infrastructure, and a server that controls transmission and reception of data and thereby receive data on the testing and analysis executed by the modularized systems at Level 0 or the systems at Level 0 and Level 1.

Furthermore, the systems at Level 1 and Level 2 include the PLC with functions at Level 1 and Level 2 that are integrated and a human machine interface (HMI) for putting together pieces of information collected and transmitted from the PLC and manually operating the entire systems.

In recent years, operation of further stabilized water treatment systems has been achieved by further evolving human control via the HMI and introducing a system with an artificial intelligence (AI) engine using machine learning (ML) mounted thereon as the systems at Level 3 and Level 3.5 that are much higher levels. In this case, data historian is provided in interfaces between the systems at Level 1 and Level 2 and the systems at Level 3 and Level 3.5. Control on the entire water treatment system by AI instead of a human has been achieved by causing AI to learn control that has relied on human experiences via the data historian.

As described above, terminal devices (system at Level 0) of the water treatment system 200 serving as the reference technique are put together in stages gradually to a system at a higher level constitutively and also as input/output data. In the water treatment system 200, eventually, an AI engine simulates human thought and derives an optimized solution and, using the solution, sends a command to each device (system at Level 0).

The water treatment system 200 described above, however, includes systems at Level 1, Level 2, and lower. These systems are not designed based on an idea of automated operation using AI. Before development of AI by ML, hierarchical systems obtained by putting together complicated systems in stages have been used such that a computer or a human can manage the systems. That is, a signal and behavior of a Level-0 terminal device are received to enable a grasp and management of a state by a human or data analysis, items to be managed are narrowed down, and control measures using feedback and the like have been proposed. In order to perform optimization by AI using ML in the entire water treatment system, a control loop for each hierarchy has to be optimized, and each device is not comprehensively controlled and overall optimization has been not achieved.

Furthermore, due to the hierarchization, it is far from optimization of the entire water treatment system in consideration of a combination of low-level systems, that is, Level-0 devices and expansion and replacement across hierarchies of devices. Furthermore, also from the viewpoint of cooperation of a plurality of water treatment systems, data used for AI to learn and analyze operation records in other facilities is not sufficiently accumulated because data diversion between devices is difficult. Apparatuses using a simulator have been proposed as operation support apparatuses in water treatment systems (facilities) and these apparatuses are merely simulators simulating actual equipment and thus it is hard to say that the apparatuses are ones obtained by optimizing the actual equipment as a whole.

In view of the problems of the water treatment system 200 that is used recently, in the first embodiment, a water treatment system 1 that enables optimal automated operation of the water treatment system by controlling a water treatment process from the outside of actual equipment for the edge computer 40 by using machine learning, simulation, virtualizing technology, and the like will be described.

### Functional Configuration of Water Treatment System 1

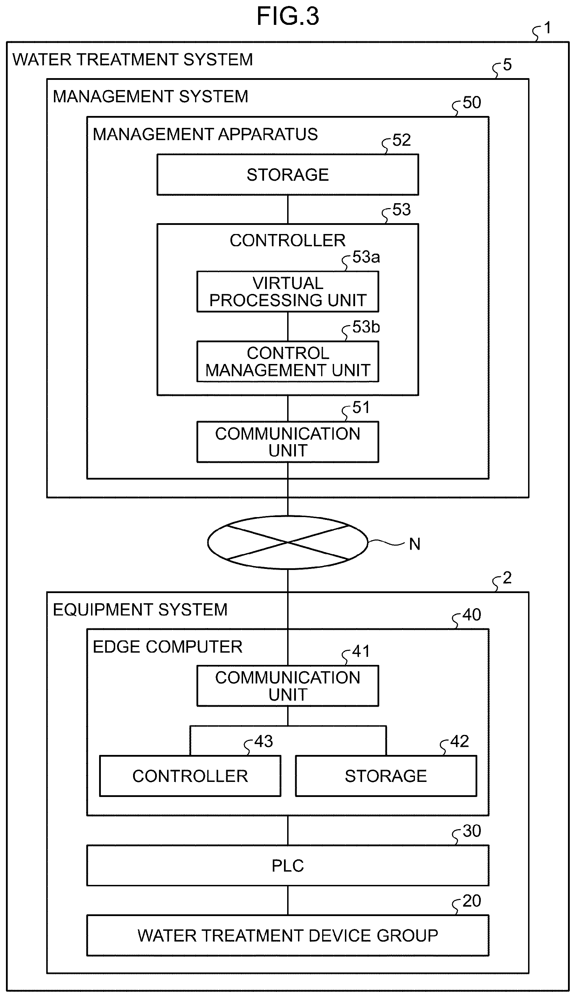

FIG. 3 is a functional block diagram illustrating a functional configuration of the water treatment system 1 according to the first embodiment. As illustrated in FIG. 3, the water treatment system 1 includes the equipment system 2 and the management system 5.

### Configuration of Equipment System 2

The equipment system 2 is a system that executes a water treatment process and includes the water treatment device group 20, the PLC 30, and the edge computer 40. The equipment system 2 does not have a hierarchized functional system configuration but constitutes a module that performs minimum operation control including individual water treatment devices and the edge computer 40. This module can also be arranged and combined so as to be directly controlled by the management system 5 (plant-level-system and software).

The water treatment device group 20 includes water treatment devices that execute water treatment processes including filtration, disinfection, piping and measurement. For example, the water treatment device group 20 includes membranes UF-RO 20a, UVAOP Ozone 20b, the mechanical components 20c, and the control H&S 20d. Note that the individual water treatment devices can be freely arranged in a module by, for example, combining the devices in parallel or in series, or arranging a plurality of devices. Moreover, the module may include not only the water treatment device but also an analysis device capable of performing sampling and analyzing water quality and performance.

The PLC 30 is an example of an apparatus that executes various instructions and sequence control to each device in the water treatment device group 20. For example, the PLC 30 executes various types of control related to the water treatment process, such as changing water temperature, open-close control on a valve, and controlling the amount of water, on each water treatment device in accordance with a logic including control (control logic) such as PID predetermined in an initial stage such as a design stage and an operation start stage of the water treatment system 1 and then the PLC 30 transmits the result (device information) to the edge computer 40.

Furthermore, the PLC 30 executes modification, addition, change, and deletion of a logic and the like in accordance with an instruction from the edge computer 40 located at a higher level. The PLC 30 then executes various types of control, such as changing the water temperature, open-close control on a valve, and control on the amount of water, in accordance with the updated logic and the like.

The edge computer 40 is an example of an apparatus that executes operation control on the water treatment process in the equipment system 2 based on a result of operation of each water treatment device executed in units of control on the water treatment process. The edge computer 40 includes a communication unit 41, a storage 42, and a controller 43.

The communication unit 41 is a processing unit that controls communication with other apparatuses and is implemented by, for example, a communication interface and the like. For example, the communication unit 41 receives data on a water treatment process such as a state and a control result from each water treatment device in the water treatment device group 20 and receives various types of data from the management apparatus 50. The communication unit 41 transmits various types of data including a control instruction to the PLC 30 and transmits various types of data on the water treatment process and various types of data on the control of the PLC 30 to the management apparatus 50.

The storage 42 is an example of a processing unit that stores various types of data and programs executed by the controller 43, and the like, and is implemented by, for example, a memory, a hard disk, and the like.

The controller 43 is a processing unit that controls the entire edge computer 40 and is implemented by, for example, a processor. Specifically, using device information on the operation status of each water treatment device in the equipment system 2 to which the controller 43 belongs, the controller 43 executes optimization of the water treatment process in the equipment system 2 to which the controller 43 belongs.

Examples of the device information include information indicating whether or not each water treatment device is normally operating and information including the content of treatment based on current set values (e.g., amount of water, temperature, and open/close level of valve) for each water treatment device and the content of control about which an instruction has been given from the PLC 30 for each water treatment device.

Then, the controller 43 generates a first condition related to the water treatment process in the equipment system 2 based on each piece of device information. Thereafter, the controller 43 outputs processing execution, processing change, and the like to the PLC 30 in accordance with the first condition. Note that examples of the first condition include an operation condition that can be controlled in the equipment system 2, such as water quality, time of disinfection, and a volume of production, within a range of a specification according to a condition of a client in the design stage of the equipment system 2, and the like.

In a specific example, the controller 43 acquires the status of the water treatment process, the operation status of each water treatment device, and the like from each water treatment device. The controller 43 acquires, from the PLC 30, a result of execution of a logic and the like performed by the PLC 30. Then, using each piece of acquired data, the controller 43 executes simulation or prediction using a machine learning model and acquires a prediction result of a preliminarily specified item such as a state, a risk level, and a cost of the water treatment process. Thereafter, the controller 43 executes changing the content of control and the like for the PLC 30 in accordance with the prediction result.

More specifically, the controller 43 increases the opening degree of a valve in order to increase a volume of production at the time when the volume of production is predicted to decrease or activates a temperature adjustment device in which alarm output of abnormally high temperature is predicted after several hours.

In other words, the controller 43 (edge computer 40) is able to execute control for the optimized operation of the water treatment system 1 in order to maintain the volume of production, reduce costs, and perform stable operation within a range of a specification predetermined at the time of designing the inside of the equipment system 2.

The controller 43 is also able to change or add a logic and the like of the PLC 30 by executing optimization of the water treatment process in the equipment system 2, to which the controller 43 belongs, in accordance with an instruction from the management system 5 (plant-level-system and software). For example, the controller 43 adds a logic and the like that increase an amount of water for increasing the volume of production, changes a threshold of an abnormality in temperature, which has been set in an existing logic and the like, to a new threshold newly set on the side of the management system 5, and reduces a part of the logic and the like for reducing costs.

In other words, the controller 43 (edge computer 40) can also execute control for optimized operation of the water treatment system 1 beyond the range of the specification predetermined at the time of designing the inside of the equipment system 2 in accordance with an instruction of the management system 5 (plant-level-system and software). This is, however, merely one example, and the instruction from the management system 5 (plant-level-system and software) may be within the range of the specification predetermined at the time of designing the inside of the equipment system 2.

### Configuration of Management System 5

As illustrated in FIG. 3, the management system 5 includes the management apparatus 50 implemented by a physical machine or a virtual machine. The management apparatus 50 includes a communication unit 51, a storage 52, and a controller 53.

The communication unit 51 is a processing unit that controls communication with other apparatuses and is implemented by, for example, a communication interface and the like. For example, the communication unit 51 receives, from the edge computer 40, content of control by the PLC 30 and various types of data generated in the equipment system 2. The communication unit 51 transmits various types of data generated by the controller 53 to the edge computer 40.

The storage 52 is an example of a processing unit that stores various types of data, programs executed by the controller 53, and the like, and is implemented by, for example, a memory and a processor.

The controller 53 is a processing unit that controls the entire management apparatus 50 and is implemented by, for example, the processor. Specifically, the controller 53 includes a virtual processing unit 53a and a control management unit 53b and executes operation control on the water treatment process in the equipment system 2 via the edge computer 40.

The virtual processing unit 53a is a processing unit that acquires data on the operation status of the water treatment process in the equipment system 2 from the edge computer 40 and that, using each water treatment device virtualized by using the virtualizing technology and the acquired data, executes simulation of the water treatment process. That is, the virtual processing unit 53a corresponds to the digital twin 50a in FIG. 1 and virtually configures a virtual system that simulates the same variation as that of the equipment system 2, which is actual equipment.

The control management unit 53b is a processing unit that executes control on a preprocessing process in the equipment system 2 via the edge computer 40. Specifically, the control management unit 53b corresponds to the AI engine 50b in FIG. 1 and, when, for example, a change in the specification of the equipment system 2, a change in the request, or a change in the demand of the total amount of generated water occurs, the control management unit 53b executes simulation using the virtual processing unit 53a. Then, based on the result of the simulation, the control management unit 53b executes a change in a logic currently executed in the PLC 30 and the like.

For example, the control management unit 53b acquires process information on the operation status of the water treatment process from the edge computer 40 of the equipment system 2 and generates content of control that optimizes the entire equipment system 2 based on the acquired process information. The control management unit 53b then outputs the content of control to the edge computer 40 of the equipment system 2. Note that examples of the process information include the content of control about which the edge computer 40 has given an instruction to the PLC 30, the execution status of the water treatment process executed according to the content of control, and the content of simulation executed by the virtual processing unit 53a (digital twin 50a).

In an example of the optimization, using a trained machine learning model and the like, the control management unit 53b is able to execute various types of prediction related to the water treatment process in the equipment system 2. For example, using various types of data acquired, generated, and simulated by the virtual processing unit 53a (digital twin 50a), the control management unit 53b generates a second condition related to the water treatment process. Then, the control management unit 53b notifies the edge computer 40 of the content of control in accordance with the second condition. As a result, the edge computer 40 executes a change and the like in the content of control via the PLC 30.

Note that the second condition includes the content of operation and the like in accordance with information, which has been, for example, specified from the outside of the equipment system 2. For example, the control management unit 53b executes simulation by using the virtual processing unit 53a (digital twin 50a) when a change in the specification of a client occurs or improvement including costs and the like is proposed to the client outside the range of the specification of the equipment system 2. Thereafter, when the simulation result is permitted by the client or the like, the control management unit 53b outputs an instruction to change a logic and the like, the logic after the change, and the like to the edge computer 40 in accordance with the result of the simulation.

### Configuration of Measurement Apparatus

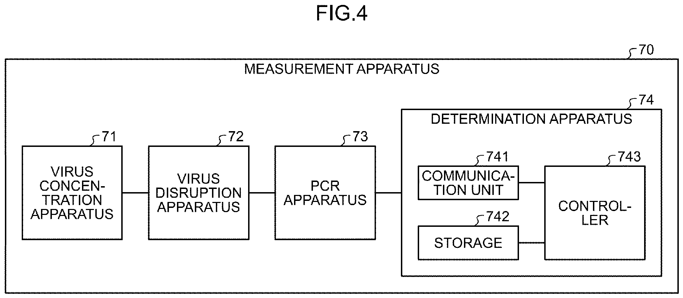

Using FIG. 4, a configuration of a measurement apparatus 70 will be described. The measurement apparatus 70 functions as the control H&S 20d. For example, the control H&S 20d in FIG. 1 may be replaced with the measurement apparatus 70.

The advanced testing 20h has functions of measurement related to management of water quality safe to the human body among operation quality management and transmission and transfer of an analog or digital signal.

A demand for visualizing an acute toxicity index such as pathogenic microorganisms like underwater viruses, bacteria, and protozoans has been increasing for reuse of sewage. Particularly against water quality having an effect on the human body, more speedy measurement, visualization of risks, and a request to optimize operation of the filtration systems 20e and the disinfection systems 20f for risk reduction and attenuation are required. The conventional technique however does not enable real-time measurement of pathogenic microorganisms and thus does not enable reflection in visualization in and operation optimization of the water treatment system based on information on the measurement.

The measurement apparatus 70 acquires qualitative information on water quality safe to the human body as data serving as a subject of machine learning by any of or any one of PCR (Polymerase Chain Reaction), TOC (Total Organic Carbon), and ATP (Adenosine Tri-Phosphate).

Especially, PCR is more valuable than TOC and ATP because PCR enables quantitation of a management index (underwater viruses themselves) necessary to grasp performance of the filtration systems 20e and the disinfection systems 20f such as blocking performance relating to acute toxicity like a rejection ratio of pathogenic microorganisms including viruses and low virulence.

Using FIG. 4, a configuration of the measurement apparatus 70 will be described. FIG. 4 is a functional block diagram illustrating a functional configuration of the measurement apparatus according to the first embodiment.

As illustrated in FIG. 4, the measurement apparatus 70 includes a virus concentration apparatus 71, a virus disruption apparatus 72, a PCR apparatus 73, and a determination apparatus 74. First of all, water that is a subject of measurement is injected into the virus concentration apparatus 71. For example, the water that is the subject of measurement may be supplied water that is supplied to the filtration systems 20e and the disinfection systems 20f or treated water that is treated by the filtration systems 20e and the disinfection systems 20f.

The virus concentration apparatus 71 concentrates viruses in the injected water. For example, the virus concentration apparatus 71 may be a concentrating pipette apparatus from InnovaPrep (trademark) (refer to CONCENTRATING PIPETTE SELECT, INNOVAPREP (URL:https://www.innovaprep.com/products/concentrating-pipette)).

The virus disruption apparatus 72 performs treatment of disrupting viruses and eluting nucleic acids on the water in which the viruses are concentrated by the virus concentration apparatus 71. For example, the virus disruption apparatus 72 elutes nucleic acids by the method described in Japanese Laid-open Patent Publication No. 2012-157265, that is, HTP (High Temperature Pressure method).

Here, it is estimated that treating the water containing viruses at high temperature and high pressure in HTP causes the viruses to be dissolved and it is expected that nucleic acids stored in the viruses are also eluted into water simultaneously. This achieves elution of nucleic acids derived from viruses from the viruses.

Japanese Laid-open Patent Publication No. 2012-157265 describes that nucleic acid elution from mold and bacteria is performed by HTP. In the present embodiment, using a protocol (a parameter setting) suitable for nucleic acid elution from viruses, the virus disruption apparatus 72 is caused to perform HTP to perform nucleic acid elution from viruses.

In other words, the virus disruption apparatus 72 guides the water that is the subject of measurement into a container, seals the container, and heats the water in the container for less than a given time to a given temperature at or above 100°C with the container being sealed.

A given maximum temperature and a given time are set as parameters of HTP. For example, the given temperature is within a range between 120°C and 160°C and the given time is within a range between 5 seconds and 30 seconds. Particularly, the given temperature may be 140°C and the given time may be 15 seconds.

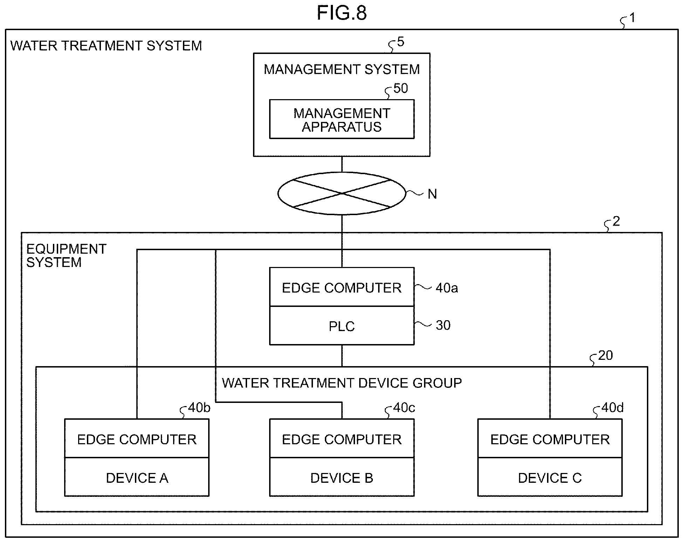

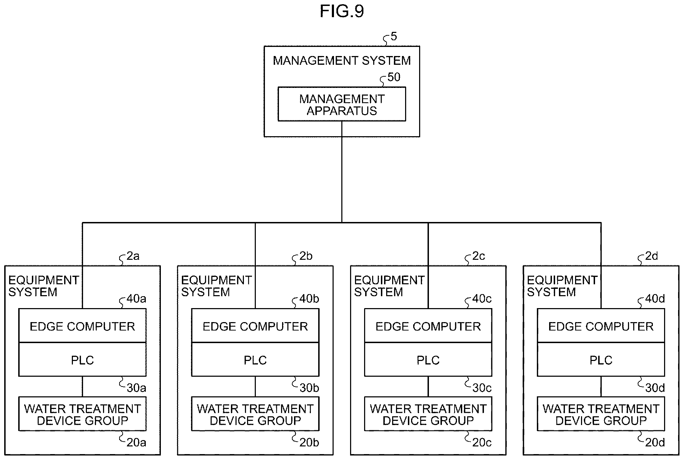

The given maximum temperature and the given time may be determined based on a result of measuring PMMoV (Pepper Mild Mottle Virus) at the time when the temperature of heating and the time of heating of HTP is changed. Examples of the result of the measurement are illustrated in FIG. 8 and FIG. 9. For example, a temperature of heating and a time of heating by which a detected amount of PMMoV increases as much as possible may be set for the given maximum temperature and the given time.

For example, the amount of PMMoV that is detected at the temperature of heating within the range between 120°C and 160°C. For example, the amount of PMMoV that is detected for a time of heating within a range between 5 seconds and 30 seconds (particularly around 15 seconds) increases sufficiently.

The PCR apparatus 73 performs 1-step RT-qPCR on the nucleic acid eluted by the virus disruption apparatus 72 and measures a quantity of viruses.

2-step RT-qPCR includes two steps of cDNA synthesis (reverse transcription) and quantitation by qPCR. Thus, 2-step RT-qPCR is advantageous in that cDNA can be stored for a long period and it is effective in screening. On the other hand, 2-step RT-qPCR is disadvantageous in requiring a long time.

1-step RT-qPCR, on the contrary, is advantageous in requiring a shorter time than 2-step RT-qPCR.

In the present embodiment, because the nucleic acids have been eluted by the virus disruption apparatus 72, a time-consuming operation such as column work used with an RNA elution kit in 2-step RT-qPCR is unnecessary. As a result, it is possible to perform virus measurement in the water treatment system 1 in a short time.

For example, 1-step RT-qPCR is implemented by treating water containing viruses by HTP with a tube dedicated to HTP. First of all, the PCR apparatus 73 previously holds a tube in which a reaction solution (a primer, a probe, and enzyme for RT-qPCR) containing transcriptase necessary for reverse transcription and polymerase necessary for qPCR is stored in liquid form or in frozen and dried form.

The PCR apparatus 73 dispenses a sample (water containing the eluted nucleic acids, that is, nucleic acids derived from viruses) received from the virus disruption apparatus 72 into the reaction solution in liquid form or in frozen and dried form in the tube, mixes the solution uniformly, and thereafter performs qPCR using the real time PCR apparatus.

The PCR apparatus 73 is also able to hold a plurality of tubes with a reaction solution therein, add, for example, a plurality of virus-concentrated solutions in which concentrations of viruses are adjusted according to standards of PMMoV to the tubes, respectively, and perform absolute quantitation. The standards of PMMoV in the respective tubes are, for example, the following nine standards. $1.0 \times {10}^{8} gene copies / μ 1$ $1.0 \times {10}^{7} gene copies / μ 1$ $1.0 \times {10}^{6} gene copies / μ 1$ $1.0 \times {10}^{5} gene copies / μ 1$ $1.0 \times {10}^{4} gene copies / μ 1$ $1.0 \times {10}^{3} gene copies / μ 1$ $1.0 \times {10}^{2} gene copies / μ 1$ $1.0 \times {10}^{1} gene copies / μ 1$ $1.0 \times {10}^{0} gene copies / μ 1$

The measurement apparatus 70 has a housing. In the housing, the virus concentration apparatus 71, the virus disruption apparatus 72, the PCR apparatus 73, and the determination apparatus 74 are stored. In other words, the measurement apparatus 70 is a package of the apparatuses. Thus, the measurement apparatus 70 is portable. Note that the measurement apparatus 70 can be assembled. In other words, each apparatus can be installed in and uninstalled from the housing.

The measurement apparatus 70 that is packaged is can be installed in and uninstalled from the water treatment system 1. The measurement apparatus 70 only has to have an interface for connection enabling data communication with other apparatuses and an inlet for taking water in.

The determination apparatus 74 determines whether or not to perform maintenance of filtration membranes based on the result of the measurement by the PCR apparatus 73.

As illustrated in FIG. 4, the determination apparatus 74 includes a communication unit 741, a storage 742, and a controller 743.

The communication unit 741 is a processing unit that controls communication with other apparatuses and is implemented by, for example, a communication interface and the like.

The storage 742 is an example of a processing unit that stores various types of data and programs executed by the controller 743, and the like, and is implemented by, for example, a memory, a hard disk, and the like.

The controller 743 is a processing unit that controls the entire determination apparatus 74 and is implemented by, for example, a processor.

The controller 743 controls the entire determination apparatus 74. The controller 743 includes an acquisition unit 6431 and an evaluator 6432. The controller 743 can be implemented by, for example, an electric circuit, such as a CPU or a MPU, and an integrated circuit, such as an ASI or a FPGA.

The controller 743 acquires a first quantity that is a quantity of viruses in membrane filtration supplied water that is supplied to a membrane filtration apparatus and a second quantity that is a quantity of viruses in membrane filtration permeated water after membrane filtration performed by the membrane filtration apparatus. In other words, the controller 743 acquires the first quality and the second quantity that are measured by a step of heating water that is a subject of measurement to a given temperature for less than a given time to a given temperature, a step of eluting nucleic acids of viruses contained in the heated water, and a step of performing 1-step RT-qPCR on the water from which the nucleic acids have been eluted. The controller 743 is able to acquire the first quantity and the second quantity from the PCR apparatus 73 as a measurement result.

The controller 743 determines whether or not to perform maintenance of the membrane filtration apparatus based on the first quantity and the second quantity.

For example, the controller 743 determines whether or not to perform maintenance of the membrane filtration apparatus based on a ratio of microorganisms that are separated by the membrane filtration apparatus based on the first quantity and the second quantity (for example, a value obtained by dividing the first quantity by the second quantity).

A physical condition such as dirt on membranes and damage of membranes is conventionally grasped by calculating information such as a water pressure and an amount of water and is used to optimize necessity of cleaning and replacement of membranes. On the other hand, when membranes are cleaned excessively, while dirt on the membranes is eliminated, this loses an opportunity of utilizing the dirt on the membranes as natural coating and forming pores smaller than the original membrane pore diameter, thereby contributing to an increase in the rate of removal of pathogenic microorganisms including viruses. Furthermore, excessive chemical cleaning may shorten the life of membranes. To deal with this including the above-described problem, in the case where the object is to increase blocking of pathogenic microorganisms including viruses in view of separation that is the original object of membrane separation including the above-described problem, the controller 743 contributes to optimization of frequency of cleaning in view of the blocking performance.

A filtration resistance R is estimated according to Darcy's law below. In Darcy's law, the filtration resistance R is given by Equation (1) using an inter-membrane differential pressure P that is a differential of a primary and a secondary in consideration of a driving pressure (a pump pushing pressure on a membrane primary side or a suction pressure on a membrane secondary side), a permeation flux J of permeation per membrane unit area, and a viscosity (viscosity coefficient) u of a fluid per water temperature.$R = P / \left(uJ\right)$

Furthermore, the filtration resistance R is given by Equation (2) using a resistance Rm unique to membranes and a resistance Rc caused by a cake layer that is formed on the membranes by dirt or clogs.$R = Rm + Rc$

For example, in optimal operation of membrane filtration, it is considered that the Rc is calculated and a trend of a behavior of Rc is grasped and predicted and thereby appropriate cleaning is performed.

While Rc depends on dirt of a membrane surface, Rc increases the function of the cake layer in terms of removal of viruses. According to the present embodiment, it is possible to incorporate the function of the cake layer and increase or maintain the rate of removal of viruses.

Assuming that the permeation flux J is constant to a set value and setting an upper limit of the inter-membrane differential pressure P, a conventional membrane filtration system performs a backwash (cleaning by filtration water) in a filtration time of, for example, 30 minutes within a range of the filtration time between one minute and one day. For this reason, the conventional membrane filtration system generally assigns an operation sequence of filtration and cleaning in only the filtration time without any consideration of formation and growth of the cake layer. Meanwhile, cleaning is performed when Rm+Rc is around one time or double an absolute value of Rm.

On the other hand, the controller 743 calculates a filtration resistance (for example, Rc) of the membrane filtration apparatus based on the first quantity and the second quantity. The controller 743 determines to perform maintenance of the membrane filtration apparatus when a sum of the calculated filtration resistance and a resistance (for example, Rm) unique to the membrane filtration apparatus is larger than a value obtained by multiplying the resistance unique to the membrane filtration apparatus by a certain multiplier factor.

For example, when the certain multiplier factor is set at ten, the controller 743 determines to perform cleaning when Rm+Rc is 10 times Rm.

The controller 743 may determine whether or not to perform maintenance based on the result of the measurement performed by the PCR apparatus 73 by associating the result of the measurement performed by the PCR apparatus 73 with Rc. For example, the controller 743 is able to determine timing of cleaning the filtration membranes based on the result of a PCR test while visualizing that microorganism separation by the filtration membranes can be achieved with a given range (for example, between 90% and 99.999%).

For example, the controller 743 determines to perform maintenance of the membrane filtration apparatus when the ratio is out of a range determined by an upper limit and a lower limit that are determined previously. The controller 743 may determine whether or not to perform maintenance of the membrane filtration apparatus using a machine learning model based on the first quantity and the second quantity. In this case, using the first quantity and the second quantity as an input, the machine learning model may output Rc of the filtration membranes. The controller 743 makes a determination based on Rc that is output by the machine learning model.

### Flow of Process

An example of a flow of a process that the equipment system 2 executes and an example of a flow of a process that the management system 5 executes will be described next.

### Process performed by Equipment System 2

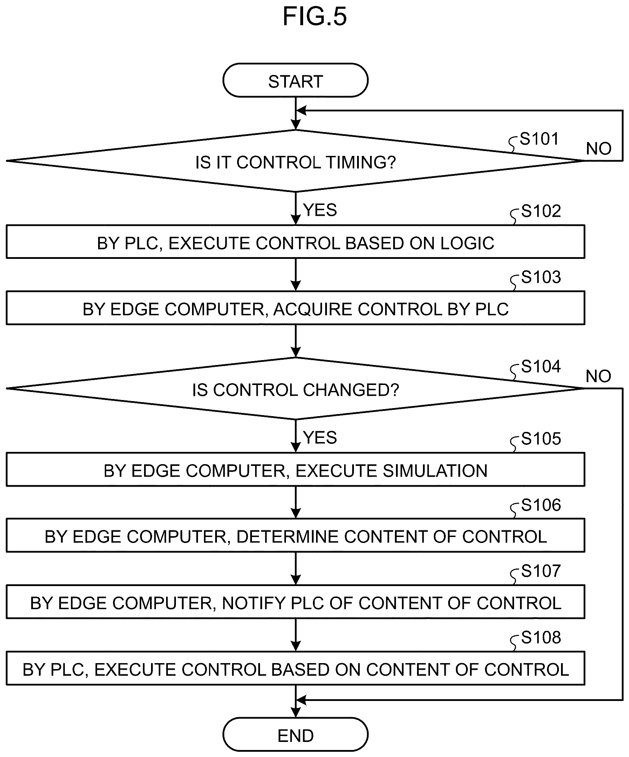

FIG. 5 is a flowchart illustrating the flow of the process executed by the equipment system 2. As illustrated in FIG. 5, when predetermined control timing is reached (S101: Yes), the PLC 30 executes control on the water treatment process based on a logic that has been set and the like (S102). The edge computer 40 then acquires the result of the control by the PLC 30 (S103), detects an abnormality, occurrence of deviation from a normal value, or the like, based on the result of the control by the PLC 30, and determines whether to change the control (S104).

On determining that the control change is necessary (S104: Yes), the edge computer 40 executes simulation and the like (S105) and determines content of control to be executed as a countermeasure (S106).

The edge computer 40 then notifies the PLC 30 of the control content (S107) and the PLC 30 makes a change and the like of the set logic and the like based on the control content of which the PLC 30 is notified and executes control on the water treatment process (S108).

### Process performed by Management System 5

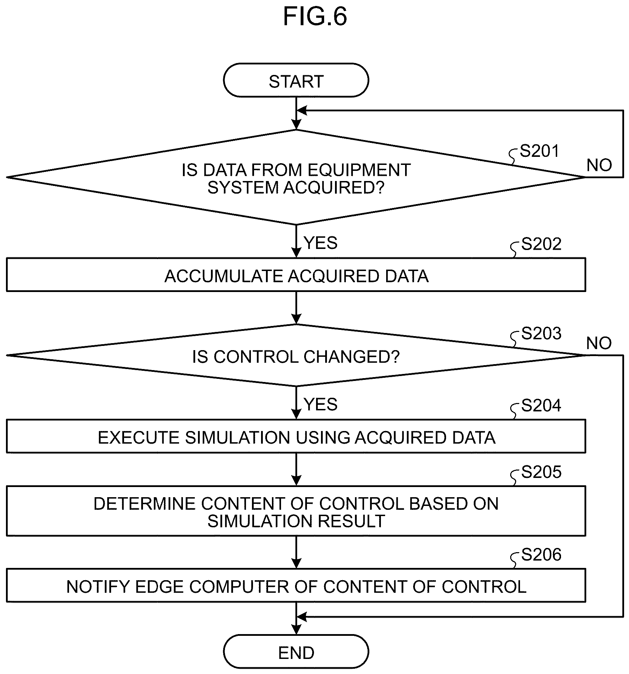

FIG. 6 is a flowchart illustrating the flow of the process executed by the management system 5. As illustrated in FIG. 6, on acquiring data of a state and a process result related to the water treatment process from the equipment system 2 via the edge computer 40 (S201: Yes), the management system 5 accumulates the acquired data (S202).

Then, when a control change including a change in the demand, a change in the setting, a change in the request, and an abnormal state occurs (S203: Yes), the management system 5 executes simulation using the acquired data and using the virtual system of the water treatment process that is generated by the virtual processing unit 53a (digital twin 50a) (S204).

Thereafter, using a table in which the simulation result is associated with a design change and the like, the management system 5 determines content of control for improving the water treatment process performed by the equipment system 2 and changing the design (S205).

The management system 5 then notifies the edge computer 40 of the equipment system 2 of the content of control (S206). As a result, the edge computer 40 executes a change in the logic of the PLC 30 and the like and the PLC 30 executes a process according to a new logic and the like, whereby a change of the water treatment process and the like are executed. Note that the management system 5 can also output the content of control generated by simulation and the like to the management apparatus, a display, and the like.

### Process performed by Controller 743

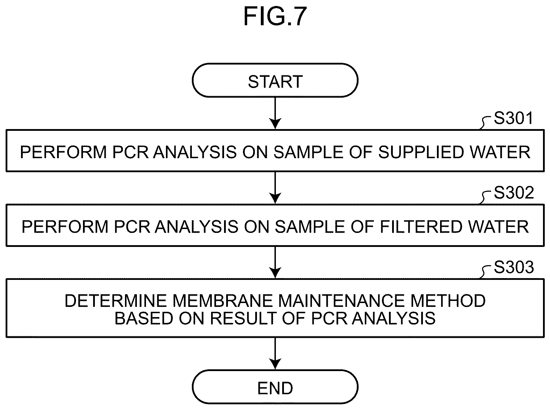

FIG. 7 is a flowchart illustrating a flow of a process that is executed by the measurement apparatus. As illustrated in FIG. 7, first of all, the PCR apparatus 73 performs PCR analysis on a sample of supplied water (step S301). The PCR apparatus 73 also performs PCR analysis on a sample of filtration water (step S302).

The controller 743 determines a method of maintenance of membranes based on the result of the PCR analysis (step S303). For example, the controller 743 determines whether or not to replace membranes or clean the membranes.

### Effects

As described above, the water treatment system 1 according to the first embodiment enables flexible expansion of a module in response to a device expansion request in accordance with a demand. The water treatment system 1 enables data collected from individual water treatment devices used in various water treatment facilities to be diverted mutually without depending on the configuration of a water treatment device and thus the water treatment system 1 enables optimal control.

The water treatment system 1 is able to instantaneously calculate a module or a combination to operate without depending on experiences and the like and automatically propose the module or a combination by using the edge computer 40 and performing management at a module level. The water treatment system 1 is able to estimate an input/output value without testing actual equipment by simulating a method for addressing a sudden environmental variation on a virtual system in equipment with a little environmental variation.

The determination apparatus 74 is also able to acquire a first quantity that is a quantity of viruses in membrane filtration supplied water that is supplied to the membrane filtration apparatus and the second quantity that is the quantity of viruses in membrane filtration permeated water after membrane filtration performed by the membrane filtration apparatus and determine whether or not to perform maintenance of the membrane filtration apparatus based on the first quantity and the second quantity. This makes it possible to grasp performance of the water treatment system 1 such as blocking performance relating to acute toxicity and low virulence. As a result, it is possible to grasp the performance of the water treatment system 1 efficiently.

### Second Embodiment

In the first embodiment, an example in which one edge computer 40 controls the entire water treatment device group 20 via the PLC 30 in the equipment system 2 has been described; however, the control is not limited to this. For example, an edge computer can be installed for each water treatment device in the water treatment device group 20 and the management system 5 can control the individual water treatment devices. Thus, in a second embodiment, an example in which the management system 5 directly controls each water treatment device and a configured module in the actual equipment will be described.

FIG. 8 is a diagram illustrating an architecture of the water treatment system 1 according to the second embodiment. As illustrated in FIG. 8, the water treatment system 1 according to the second embodiment includes the equipment system 2 and the management system 5 as in the first embodiment.

The difference from the first embodiment is that edge computers 40a, 40b, 40c, and 40d are provided for the PLC 30, and each of a device A, a device B, and a device C of the water treatment device group 20 in the equipment system 2. Note that the edge computers 40a, 40b, 40c, and 40d have a function similar to that of the edge computer 40 described in the first embodiment.

For example, the management system 5 acquires data on the state of the PLC 30 or each water treatment device, the control status, the control result, and the like from each of the edge computers 40a, 40b, 40c, and 40d. Then, the management system 5 generates content of control related to the operation of each water treatment device by using a virtual system, AI (machine learning model), and the like. The management system 5 then notifies each of the edge computers 40a, 40b, 40c, and 40d of the generated content of control.

As a result, each of the edge computers 40a, 40b, 40c, and 40d executes control in accordance with the control content of which each of the edge computers has been notified by the management system 5 for the PLC and each device. As described above, the management system 5 (AI engine 50b) directly controls the minimum unit of water treatment device in units of modules, thereby enabling more optimal control at high speed.

Furthermore, the management system 5 can obtain a solution that optimizes the entire processing system and thus optimize an operation in units of water treatment devices using each edge computer constituting a module, thereby combining modules. Furthermore, the management system 5 can directly send an instruction to the water treatment device and thereby the speed of operation to the water treatment device increases. Furthermore, it is possible to increase the degree of freedom of combination of water treatment devices, increase the degree of freedom of combination of parameters, and consider a causal relation between terminal devices. The management system 5 is able to quickly and appropriately derive a countermeasure against an unexpected variation.

### Third Embodiment

In the first embodiment and the second embodiment, the example where the single management system 5 manages and controls the single water treatment system (equipment system 2) has been described; however, the control is not limited to this. For example, the single management system 5 manages an integrated system obtained by combining a plurality of water treatment devices as a single water treatment device and thereby enables perspective control on the entire water treatment system.

FIG. 9 is a diagram illustrating an architecture of the water treatment system 1 according to a third embodiment. As illustrated in FIG. 9, the water treatment system 1 according to the third embodiment includes the management system 5 and a plurality of equipment systems 2a, 2b, 2c, and 2d.

The management system 5 has a function similar to that of the management system 5 described in the first embodiment and each of the equipment systems 2a, 2b, 2c, and 2d has a function similar to that of the equipment system 2 described in the first embodiment.

The equipment system 2a includes a water treatment device group 20a, a PLC 30a, and the edge computer 40a and the equipment system 2b includes a water treatment device group 20b, a PLC 30b, and the edge computer 40b. The equipment system 2c includes a water treatment device group 20c, a PLC 30c, and the edge computer 40c and the equipment system 2d includes a water treatment device group 20d, a PLC 30d, and the edge computer 40d.

Note that, the water treatment device groups 20a, 20b, 20c, and 20d have a configuration similar to that of the water treatment device group 20 described in the first embodiment. The PLCs 30a, 30b, 30c, and 30d have a function similar to that of the PLC 30 described in the first embodiment. The edge computers 40a, 40b, 40c, and 40d have a function similar to that of the edge computer 40 described in the first embodiment.

In such a configuration, the management system 5 collects data not only from a single water treatment system but from a plurality of water treatment systems. For example, the management system 5 acquires various types of data on the water treatment process from each of the equipment systems 2a, 2b, 2c, and 2d. Then, the management system 5 generates content of control related to an integrated operation index of a plurality of equipment systems based on the operation status and the like of the water treatment process of each of the equipment systems. Thereafter, the management system 5 executes operation control on the water treatment process executed by each edge computer of each equipment system in accordance with the content of control.

For example, a water treatment system in which each equipment system 2 is installed in the same area is assumed. In this state, even when an instruction to increase a volume of production from the entire system is given, the management system 5 is able to make a change to content of control for increasing the operation rate of the equipment system 2b having a volume of production to spare. As a result, the management system 5 makes it possible to increase the volume of production by distributing a load on the entire water treatment system and thereby avoid a risk associated with stop of any water treatment system.

In the above-described state, even when an instruction to increase the volume of production from the equipment system 2a is given, the management system 5 is able to make a change to content of control for increasing the operation rate of the equipment system 2c having the lowest operation costs. As a result, the management system 5 enables an increase in the volume of production while executing cost reduction of the entire water treatment system.

The management system 5 is able to collect and integrally manage the operation status, capital investment, costs, and the like of each equipment system 2. As a result, when an operation load on a certain equipment system is abnormally high, the management system 5 can also propose a client for using another equipment system.

Furthermore, the management system 5 manufactures the equipment system 2a and the equipment system 2b that meet a request from the client and executes the water treatment process. Thereafter, even when the need to generate a new equipment system arises in association with a change in the request from the client, the management system 5 is able to respond to the request from the client while reducing the costs of generating the new system by proposing use of the equipment system 2d having the same configuration.

As described above, the water treatment system 1 according to the third embodiment enables data collected from a plurality of water treatment systems to be mutually diverted and optimized not in association with individual water treatment systems (equipment systems).

### Fourth Embodiment

The embodiments of the present disclosure have been described; however, the disclosure may be implemented in various different modes other than the above-described embodiments.

### Numerical Value and the Like

The number of water treatment devices, the number of equipment systems, specific examples of content of control, and the like described in the above-described embodiments are examples only and they can be optionally changed. Furthermore, in the flowcharts described in the embodiment, the order of processing is changeable within a range without contradiction.

### System

Information including the process procedures, the control procedures, the specific names, and various types of data and parameters in the document above and the drawings is optionally changeable unless otherwise specified. For example, the water treatment system 1 may be configured such that actual equipment automatically simulates an optimized virtual system configuration calculated by the management system 5 (plant-level-system and software).

Furthermore, each component of each illustrated apparatus is functionally conceptual, and is not necessarily required to be physically configured as illustrated in the drawings. That is, a specific mode of distribution and integration of each apparatus is not limited to that illustrated in the drawings. That is, all or a part thereof can be functionally or physically distributed and integrated in any unit in accordance with various types of loads, the status of use, and the like.

Moreover, all or any part of each processing function performed in each apparatus can be implemented by a central processing unit (CPU) and a program analyzed and executed by the CPU or can be implemented as hardware using a wired logic.

### Hardware

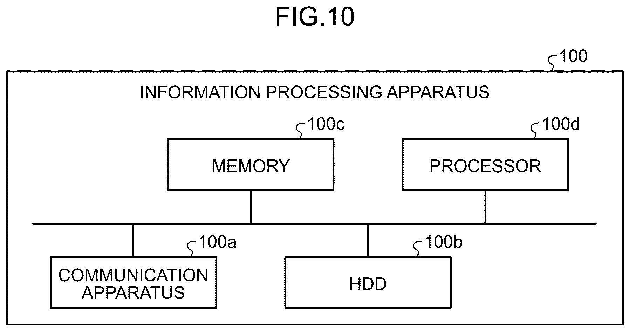

Next, an example of hardware configurations of the computers described in the embodiments will be described. Note that, since the management apparatus 50, the edge computer 40, and the determination apparatus 74 have similar hardware configurations, the management apparatus 50 and the edge computer 40 will be described as an information processing apparatus 100. FIG. 10 is a diagram illustrating an example of the hardware configuration. As illustrated in FIG. 10, the information processing apparatus 100 includes a communication apparatus 100a, a hard disk drive (HDD) 100b, a memory 100c, and a processor 100d. Furthermore, the units illustrated in FIG. 10 are connected to each other by a bus or the like.

The communication apparatus 100a is a network interface card or the like and communicates with another server. The HDD 100b stores a program for causing the functions illustrated in FIG. 3 to operate and a DB.

The processor 100d reads a program for executing processing similar to that of the processing units illustrated in FIG. 3 from the HDD 100b or the like and loads the program in the memory 100c, thereby operating a process for executing each function described with reference to FIG. 3 and the like. For example, when described by taking the management apparatus 50 as an example, the process executes a function similar to that of each processing unit of the management apparatus 50. Specifically, the processor 100d reads a program having functions similar to those of the virtual processing unit 53a, the control management unit 53b, and the like from the HDD 100b and the like. Then, the processor 100d executes a process of executing processing similar to that of the virtual processing unit 53a, the control management unit 53b, and the like.

As described above, the information processing apparatus 100 operates as an information processing apparatus that executes a determination method by reading and executing the program. The information processing apparatus 100 can also implement a function similar to those of the above-described embodiments by reading the above-described program from a recording medium with a medium reading apparatus and executing the read program. Note that the program in other embodiments is not limited to being executed by the information processing apparatus 100. For example, the present disclosure can be similarly applied even to the case where another computer or another server executes a program or the case where the computer and the server execute the program cooperatively.

The program can be distributed via a network such as the Internet. Furthermore, the program is recorded in a computer-readable recording medium such as a hard disk, a flexible disk (FD), a CD-ROM, a magneto-optical (MO) disk, or a digital versatile disc (DVD), and the program can be executed by being read from the recording medium by the computer.

Some examples of a combination of the disclosed technical features will be described below.

According to the disclosure, it is possible to grasp performance of a water treatment system efficiently.

Although the invention has been described with respect to specific embodiments for a complete and clear disclosure, the appended claims are not to be thus limited but are to be construed as embodying all modifications and alternative constructions that may occur to one skilled in the art that fairly fall within the basic teaching herein set forth.

## Claims

1. A determination apparatus (74) comprising a processor that executes
acquiring a first quantity that is a quantity of viruses in membrane filtration supplied water that is supplied to a membrane filtration apparatus and a second quantity that is a quantity of viruses in membrane filtration permeated water after membrane filtration performed by the membrane filtration apparatus, and
determining whether or not to perform maintenance of the membrane filtration apparatus based on the first quantity and the second quantity.

2. The determination apparatus (74) according to claim 1, wherein the processor determines whether or not to perform maintenance of the membrane filtration apparatus based on a ratio of microorganisms that are separated by the membrane filtration apparatus based on the first quantity and the second quantity.

3. The determination apparatus (74) according to claim 2, wherein the processor determines to perform maintenance of the membrane filtration apparatus when the ratio is out of a range that is determined by an upper limit and a lower limit that are determined previously.

4. The determination apparatus (74) according to any one of claims 1 to 3, wherein the processor acquires the first quantity and the second quantity that are measured by a step of heating water that is a subject of measurement to a given temperature for less than a given time to a given temperature, a step of eluting nucleic acids of viruses contained in the heated water, and a step of performing 1-step RT-qPCR on the water from which the nucleic acids have been eluted.

5. The determination apparatus (74) according to any one of claims 1 to 4, wherein the processor determines whether or not to perform maintenance of the membrane filtration apparatus using a machine learning model based on the first quantity and the second quantity.

6. The determination apparatus (74) according to any one of claims 1 to 5, wherein the processor calculates a filtration resistance of the membrane filtration apparatus based on the first quantity and the second quantity.

7. The determination apparatus (74) according to claim 6, wherein the processor determines to perform maintenance of the membrane filtration apparatus when a sum of the calculated filtration resistance and a resistance unique to the membrane filtration apparatus is larger than a value obtained by multiplying the resistance unique to the membrane filtration apparatus by a certain multiplier factor.

8. A determination method carried out by a computer, comprising:
acquiring a first quantity that is a quantity of viruses in membrane filtration supplied water that is supplied to a membrane filtration apparatus and a second quantity that is a quantity of viruses in membrane filtration permeated water after membrane filtration performed by the membrane filtration apparatus, and
determining whether or not to perform maintenance of the membrane filtration apparatus based on the first quantity and the second quantity.

9. A determination program that causes a computer to
acquire a first quantity that is a quantity of viruses in membrane filtration supplied water that is supplied to a membrane filtration apparatus and a second quantity that is a quantity of viruses in membrane filtration permeated water after membrane filtration performed by the membrane filtration apparatus, and
determine whether or not to perform maintenance of the membrane filtration apparatus based on the first quantity and the second quantity.
